# EUROPEAN PATENT SPECIFICATION

(11) **EP 1 552 854 B1**
(45) Date of publication and mention of the grant of the patent: **04.04.2007**
(21) Application number: 02785423.1
(22) Date of filing: 15.10.2002
(51) Int. Cl.: A61L 9/02, A61L 9/03

(54) **METHOD OF DISINFECTING, FRESHENING AND SCENTING THE AIR USING ESSENTIAL OILS AND/OR THE ACTIVE PRINCIPLES THEREOF**
VERFAHREN ZUM DESINFIZIEREN, ERFRISCHEN UND PARFÜMIEREN VON LUFT MIT ÄTHERISCHEN ÖLEN UND/ODER DEREN WIRKSTOFFEN
PROCEDE DE DESINFECTION, DE DESODORISATION ET D'AROMATISATION DE L'AIR AU MOYEN D'HUILES ESSENTIELLES ET/OU DE LEURS PRINCIPES ACTIFS

(43) Date of publication of application: 13.07.2005
(73) Proprietor: Zobele Espana, S.A., 08290 Cerdanyola del Vallés (ES); Sensient Fragrances S.A., Armilla, 18100 Granada (ES)
(72) Inventor: CORTES BAREA, Jordi, E-08290 Cerdanyola del Valles (ES); RUIZ SANCHEZ, Antonio, SENSIENT FRAGRANCES, S.A., Armilla, 18100 Granada (ES); CASERTA, Andrea, E-08290 Cerdanyola del Vallés (ES); GARCIA FABREGA, Ruben, E-08290 Cerdanyola del Vallés (ES)
(74) Representative: Carpintero Lopez, Francisco
(86) International application number: PCT/ES2002/000486
(87) International publication number: WO 2004/035097

(56) References cited:
- WO-A-01/76371
- FR-A- 2 565 109
- US-A- 4 663 315
- US-A- 4 892 711
- US-A- 5 038 394
- US-A- 5 591 395

## Description

### OBJECT OF THE INVENTION

The present invention relates to a method of disinfecting, freshening and perfuming the air based on the emission of particles into the air of a disinfecting and aromatic composition based on essential oils and/or the active ingredients thereof, wherein the particles are produced by means of evaporation through a wick indirectly heated in the upper area.

### BACKGROUND OF THE INVENTION

It is well known that the air in an enclosed space is colonized by bacterial flora of diverse nature. On numerous occasions, said enclosed spaces remain closed for hours or have a deficient ventilation and renovation of air, so that the existing bacterial load can become infectious. A large number of microorganisms uses air as a propagation medium, affecting individuals and residents in said spaces through the respiratory tract.

The present invention is directly related with the PCT application WO 03/043667. This application describes a method of disinfecting, freshening and perfuming the air based on essential oils and/or the active ingredients thereof, which consists in producing an emission of particles into the air of a disinfecting, freshening and perfuming composition which comprises essential oils and/or the active ingredients thereof, and in that the particles of the disinfecting composition are produced by means of evaporation through a wick indirectly heated in its upper area. The temperatures reached by said upper area of the wick are equal to or greater than 100° C.

Moreover, systems are known to feed insecticides into the air through producing the evaporation of an insecticide liquid through an absorbent wick immersed in the liquid itself, so that by heating the wick the evaporation of the active ingredient of the insecticide liquid takes place into the atmosphere. In the US patent 4745705 a method is described of feeding insecticides into the air based on the aforementioned principle.

Likewise, mention can be made of the US Patents 5095647; 4663315; 5038394 and 5290546, which describe systems for the feeding of insecticides into the air without describing the devices employed in the generation of particles.

Furthermore, in the British Patent GB A2 194442 a fumigator device is described capable of transpiring a chemical agent of insecticidal, disinfecting, deodorant, etc., action through a porous wick and indirect heating for the purpose of killing insects, germs or deodorizing, although said patent does not describe explicitly the method of disinfection nor the compositions for the application of the corresponding chemical agent.

In US Patent 5591395 a method is described for disinfection of the air by means of the use of particle generators for the release of disinfecting agents into the atmosphere, so that in this patent 90% of the particles emitted have a size limited to 5 microns, being likewise equally limited the range of working temperature, between 50 and 120° C.

Other documents and patents are known that concern the disinfection of surfaces through liquid formulations or aerosol generators, although the products are not disclosed for disinfection of the air, and in such sense mention can be made of the methods and/or formulations of disinfecting action which employ essential oils as active ingredients, disclosed in the Spanish Patents ES 2023530 and ES 2143172, as well as in EP 842604 A1 and WO96/39826.

### DESCRIPTION OF THE INVENTION

The main aspect of the present invention consists of a method of disinfecting, freshening and perfuming the air based on essential oils and/or the active ingredients thereof, as defined in claim 1.

By means of the referenced method of the invention a high mortality is achieved of certain microorganisms dispersed in the environmental air of a room, which can be pathogenic or transmit infections, or achieve the inhibition of microbial growth in the actual environmental air together with a freshening or perfuming of the air.

The method of disinfecting, freshening and perfuming finds special application in the household and institutional field, for disinfecting, freshening and perfuming the air in sitting rooms, bedrooms or other enclosures or closed spaces, so that the use of essential oils and/or the active ingredients thereof, as disinfecting and aromatic additives and the mixtures thereof with glycol ethers in variety of proportions, offer the following advantage with regard to other conventional methods of disinfection:
- The safety profile of essential oils and/or the active ingredients thereof is much superior to that of the majority of non-natural antimicrobial additives. In fact, the majority of essential oils of interest are accepted as aromatic agents, natural repellants, food additives or natural preservatives for human or animal consumption.
- The essential oils and/or the active ingredients thereof possess intrinsic odourous properties, whereby they provide or enhance the smell of the disinfecting product, allowing the composition thereof to have freshening or aromatic properties without the necessity to add an additional perfume.

In the context of the present invention essential oil is understood to be those volatile organic substances of botanical origin constituents of plants, trees and shrubs. Essential oils can be obtained from cinnamon, the tea tree, citronella, lemongrass, thyme, citrus, anisette, cloves, geranium, rose, mint, lavender, eucalyptus, peppermint, camphor, sandalwood, cedarwood and mixtures thereof, without essential oils being limited to those obtained from the aforementioned products.

For its part, within the context of the present invention, the active ingredient of essential oils is understood to be those chemical compounds which present as particularity a proven activity in the ambit of disinfection. The active ingredients of essential oils are preferably terpineol, cinnamaldehyde, cinnamic acid, thymol, citronellal, eugenol, menthol, geraniol, eucalyptol (cineol), cedrol, anethol, cajeputene, carbacrol and mixtures thereof, without the active agents of interest being limited to these ingredients.

In a preferred manner, the essential oils for use in the present invention, are the oils of the tea tree, cinnamon, citronella, artemisia, lemongrass, cedar, cloves, pine, bergamot and lavandino, whilst the main active agents are thymol, citronellal, citronellol, estragol, geranyl acetate, eucalyptol, eugenol, terpineol, cinnamaldehyde, cinnamic acid, citral, dihydromyrcenol, rose oxide and cajeputene.

As for the glycols employed as solvents for said essential oils and/or their active agents, they will be glycol ethers.

Although the effectiveness is known of the glycols as air disinfecting agents, as is mentioned in the US patent 5591395, it is fitting to point out that the use of essential oils in the process of air disinfection and mixtures thereof with glycol ethers offers a clear advantage ver the use of only glycols as disinfecting agents. In this sense, there are numerous bibliographical studies which demonstrate that the disinfecting action of glycols is highly dependent upon ambient conditions of temperature and relative humidity. Thus, humidities greater than 35-40% reduce and can even suppress their disinfecting effectiveness. This is due to the good hygroscopic properties of glycols, which produces their condensation, minimizing the effective amount of agent in the vapour state in the air.

The aforementioned bibliographical studies, correspond to:
1) Jennings, B.H. et al. (1944) *The uses of glycol vapors for air sterilization and control of airborne infection.*
2) Robertson, O.H. (1943) *Sterilization of air with glycol vapors .*
3) Lester, William et al. (1949) *The rate of bactericidal action of triethylene glycol vapor on microorganisms dispersed into the air in small droplets.*
4) Hamburger, Morton et al. (1945) *The present status of glycol vapors in air sterilization.*
5) *Bigg, Edward et al. (1945) Epidemiologic observations on the uses of glycol vapors for air sterilization.*
6) Puck, Theodore T. et al. (1943) *The bactericidal action of propylene glycol vapor on microorganisms in air.*

The disinfecting effectiveness of essential oils and/or the active ingredients thereof or the mixtures thereof with glycols are not dependent on the conditions of temperature and relative humidity, since said agents have scant or no hygroscopy, remaining therefore in the vapour phase and as active agents for the disinfection of the air.

In a particular embodiment of the invention, the disinfecting composition, besides essential oils and/or the active ingredients thereof, as well as glycol ethers, includes aromatic compounds which do not affect the disinfecting activity of the product and their function is solely that of contributing a more pleasant and appropriate odour to the products. In the context of the present invention therefore aromatic compound is understood to be those substances which have odorising properties. The aromatic compounds employed in the present invention include, although they are not limited to chemical compounds like aldehydes (phenylacetic, C8, C9, C10, etc.), acetates (of benzyl, hexyl, octyl, etc.), ionones (gamma-methyl, beta, etc.), lactones (gamma undecalactone, decalactone, nonalactone, dodecalactone, etc.), alcohols (phenylethyl, phenylpropyl), pyrazines, or acids.

The emission of the disinfecting and aromatic particles into the air will be carried out through the evaporation of the disinfecting and aromatic liquid by means of indirect heating of the upper part of a porous wick immersed in the liquid, it being possible for the dispersion system to consist of an external cell or container with an opening in its upper part, which cell will contain the disinfecting and aromatic liquid in its interior and will allow the collocation of a porous or absorbent wick to conduct the liquid to the exterior, so that surrounding the opening of the cell is a heater element, which is limited in order not to reach temperatures of 100° C or higher, and which can be an one of the conventional heater elements, such as heater rings or one or several PTC.

In any case, the heater element will be connected by means of a conductor to a source of electric current, which can be a battery, a cell or a household connection to the electricity mains.

For its part, the absorbent wick can be of any conventional material of those employed for such a purpose, being possible preferentially ceramic materials, or based on polyester or polyethylene fibres, compacted wood, sintered plastics and even carbon fibres.

According to the present invention the wick is heated indirectly to a temperature of less than 100° C in its upper area. Working at these temperatures, a very effective and satisfactory operation is achieved of the method of disinfecting, freshening and perfuming, moreover, the total or partial degradation of some essential oils and/or the active ingredients thereof is avoided. Thus, as well as enhancing the effectiveness of the method of desinfectating, freshening and perfuming, the variety of essential oils and/or active ingredients that can be used in the method of the invention is more extensive than in the conventional methods.

A device suitable to carry out the method described above comprises a container that comes with an opening in its upper part and another in the lower part, this last one having a porous or absorbent wick to conduct the liquid from the container to the exterior, so that surrounding this opening of the container is a heater element, which is designed in such a way that it only reaches temperatures of less than 100° C.

Evidently, the effectiveness of disinfecting the air is determined by evaluation of the decrease in the microbial pollution with respect to the initial situation after having used the disinfecting product.

Experiments have been carried out using different types of formulation of the essential oil and/or its active ingredients, the references made, among others, being summarized in the following examples:

### EXAMPLE 1

The formulation tested in this case contains 40% of active ingredients, 10% of inactive aromatic ingredients and 50% of glycol ethers as solvents. Specifically, the active components which it contains are:

| **COMPONENTS** | **% IN FORMULA** |
|---|---|
| Caraway | 0.375 |
| Cedar | 0.15 |
| Cinnamon | 0.25 |
| Cypress | 0.275 |
| Dihydromyrcenol | 11 |
| Cloves | 4.9 |
| Dehydro methyl jasmonate | 4.25 |
| Orange | 9.75 |
| Patchouli | 1.1 |
| Sage | 1.1 |
| Mint | 0.55 |
| Absinthe | 0.05 |
| Armoise | 0.05 |
| Sandalwood | 1.1 |
| Geranium | 0.75 |
| Thyme | 3.25 |
| Verbena | 1.1 |
| TOTAL . | 40.0 |

### Procedure:

In a room of 1000 litres, between 0.12 and 0.18 grams of the disinfecting formulation are spread by means of its evaporation through wicks of polyester fibre heated indirectly in the upper part thereof with a PTC heater system of 75° C working temperature. At that point in time a controlled quantity of microbial pollution is introduced. A contact time of 30 minutes is allowed between the microorganism under study and the active ingredients dispersed in the air. On completion of that half hour, an air capture is carried out and the corresponding count is made of the number of colonies. This quantity corresponds to the microbial load in the air after the disinfecting product has acted on the initial pollution for a half hour.

The results are the following:

| MICROORGANISM | % MORTALITY |
|---|---|
| Salmonella cholerae | 100% |
| Microccocus luteus | 94% |
| Aspergillus Fumigatus | 96% |

These results reflect again an important reduction in the quantity of microorganisms present in the air, demonstrating the high disinfecting power of the formula.

### EXAMPLE 2

The formula tested in this case contains 37.5% of active ingredients, 12.5% of inactive aromatic ingredients, and 50% of glycol ethers as solvents. Specifically, the active components that it contains are:

| **COMPONENTS** | **% IN FORMULA** |
|---|---|
| Elemi | 0.5 |
| Anisette | 0.5 |
| Eucalyptus | 0.7 |
| Lavender | 6.2 |
| Petitgrain | 0.4 |
| Pine | 5 |
| Basil | 0.25 |
| Sage | 0.2 |
| Rose | 0.2 |
| Orange | 1.5 |
| Bergamot | 2.5 |
| Lime | 2 |
| Cinnamon | 0.2 |
| Cloves | 0.25 |
| Cedar | 4 |
| Lemon | 6.2 |
| Geranium | 6.2 |
| Litsea Cubeba | 0.4 |
| Mint | 0.1 |
| Thyme | 0.2 |
| TOTAL | 37.50 |

### Procedure:

The same procedure as for example 1

The results are the following:

| MICROORGANISM | % MORTALITY |
|---|---|
| Salmonella cholerae | 100% |
| Microccocus luteus | 99% |
| Aspergillus Fumigatus | 99% |

These results reflect again an important reduction in the quantity of microorganisms present in the air, showing the high disinfecting power of the formula.

### EXAMPLE 3

The formula tested in this case contains 35.0% of active ingredients, 15% of inactive aromatic ingredients, and 50% of glycol ethers as solvents. Specifically, the active components that it contains are:

| **COMPONENTS** | **% IN FORMULA** |
|---|---|
| Pine | 15 |
| Eucalyptus | 2.5 |
| Orange | 2.5 |
| Lime | 1 |
| Sage | 1 |
| Lavandin | 0.75 |
| Fir | 1 |
| Rosemary | 0.25 |
| Juniper berry | 1 |
| Linalol | 6 |
| Petitgrain | 1.5 |
| Caraway | 1 |
| Cedar | 1 |
| Bergamot | 0.25 |
| Nutmeg | 0.25 |
| TOTAL | 35.0 |

### Procedure:

The same procedure as for example 1

The results are the following:

| MICROORGANISM | % MORTALITY |
|---|---|
| Salmonella cholerae | 100% |
| Microccocus luteus | 96% |
| Aspergillus Fumigatus | 99% |

These results reflect again an important reduction in the quantity of microorganisms present in the air, showing the high disinfecting power of the formula.

It is stressed that the inactive aromatic ingredients do not affect the disinfecting activity of the product and their function is solely that of contributing a more pleasant and more appropriate odour to the products. This is demonstrated with the study of the same formulas with active ingredients and without aromatic components, as is detailed in the following examples:

### EXAMPLE 4

The formula tested in this case contains 45% of active ingredients and 55% of glycol ethers as solvents. Specifically, the active components which it contains are the same ones and in approximately equal proportion to that in example 1:

| **COMPONENTS** | **% IN FORMULA** |
|---|---|
| Caraway | 0.4 |
| Cedar | 0.25 |
| Cinnamon | 0.35 |
| Cypress | 0.3 |
| Dihydromyrcenol | 11.5 |
| Cloves | 5.5 |
| Dehydro methyl jasmonate | 4.5 |
| Orange | 10 |
| Patchouli | 1.5 |
| Sage | 1.5 |
| Mint | 1.0 |
| Absinthe | 0.1. |
| Armoise | 0.1 |
| Sandalwood | 1.5 |
| Geranium | 1.25 |
| Thyme | 3.75 |
| Verbena | 1.5 |
| TOTAL | 45.0 |

The results are the following:

| MICROORGANISM | % MORTALITY |
|---|---|
| Salmonella cholerae | 99% |
| Microccocus luteus | 95% |
| Aspergillus Fumigatus | 95% |

These results reflect again an important reduction in the quantity of microorganisms present in the air, showing the high disinfecting power of the formula in the absence of inactive aromatic agents.

### EXAMPLE 5

The formula tested in this case contains 42% of active ingredients and 58% of glycol ethers as solvents. Specifically, the active components that it contains are the same ones in approximately equal proportion to that in example 2:

| **COMPONENTS** | **% IN FORMULA** |
|---|---|
| Elemi | 0.55 |
| Anisette | 0.55 |
| Eucalyptus | 0.7 |
| Lavender | 6.2 |
| Petitgrain | 0.4 |
| Pine | 6 |
| Basil | 0.25 |
| Sage | 0.2 |
| Rose | 0.2 |
| Orange | 1.5 |
| Bergamot | 2.5 |
| Lime | 2 |
| Cinnamon | 0.2 |
| Cloves | 0.25 |
| Cedar | 5 |
| Lemon | 7.2 |
| Geranium | 7.2 |
| Litsea Cubeba | 0.4 |
| Mint | 0.1 |
| Thyme | 0.2 |
| TOTAL | 42.0 |

### Procedure:

The same protocol as for example 1.

The results are the following:

| MICROORGANISM | % MORTALITY |
|---|---|
| Salmonella cholerae | 100 % |
| Microccocus luteus | 99 % |
| Aspergillus Fumigatus | 99 % |

These results reflect again an important reduction in the quantity of microorganisms present in the air, showing the high disinfecting power of the formula in the absence of non-active aromatic agents.

### EXAMPLE 6

The formula tested in this case contains 44% of active ingredients and 56% of glycol ethers as solvents. Specifically, the active components that it contains are the same ones and in approximately equal proportion to that in example 3:

| **COMPONENT** | **% IN FORMULA** |
|---|---|
| Pine | 17 |
| Eucalyptus | 3 |
| Orange | 3 |
| Lime | 1.5 |
| Sage | 1.5 |
| Lavandin | 1.75 |
| Fir | 1.5 |
| Rosemary | 0.75 |
| Juniper berries | 1.5 |
| Linalol | 7 |
| Petitgrain | 1.5 |
| Caraway | 1.5 |
| Cedar | 1.5 |
| Bergamot | 0.5 |
| Nutmeg | 0.5 |
| TOTAL | 44.0 |

### Procedure:

The same protocol as for example 1.

The results are the following:

| MICROORGANISM | % MORTALITY |
|---|---|
| Salmonella cholerae | 100% |
| Microccocus luteus | 97% |
| Aspergillus Fumigatus | 99% |

These results reflect again an important reduction in the quantity of microorganisms present in the air, showing the high disinfecting power of the formula in the absence of inactive aromatic agents.

## Claims

1. - Method of disinfecting, freshening and perfuming the air based on essential oils and/or the active ingredients thereof, said method consisting in producing by evaporation, by means of an indirectly heated wick, an emission of particles into the air of a disinfecting, freshening and perfuming composition, which comprises essential oils and/or the active ingredients thereof and derivatives of glycols as dissolving medium of the essential oils and the active ingredients thereof, wherein the wick reaches a temperature of less than 100° C in its upper area, **characterised in that** the employed derivatives of glycols are glycol ethers.

2. - Method of disinfecting, freshening and perfuming the air according to claim 1, **characterised in that** the essential oils are obtained preferably from the tea tree, from the leaves of Ceylon cinnamon, from citronella, artemisia, lemongrass, cedar, cloves, pine, bergamot, lavandino or from mixtures thereof; and the preferably employed active ingredients are selected from among thymol, citronellal, citronellol, estragol, geranyl acetate, eucalyptol, eugenol, terpineol, cinnamaldehyde, cinnamic acid, citral, dihydromyrcenol, rose oxide, cajeputene and mixtures thereof.

3. - Method of disinfecting, freshening and perfuming the air according to previous claims, **characterised in that** the disinfecting composition includes aromatic compounds.

## Patentansprüche

1. Verfahren zum Desinfizieren, Erfrischen und Parfümieren der Luft, basierend auf ätherischen Ölen und/oder den Wirkstoffen davon, wobei das Verfahren besteht aus der Produktion durch Verdampfung durch einen indirekt beheizten Docht, einer Emission von Partikeln in die Luft einer desinfizierenden, erfrischenden und parfümierenden Zusammensetzung, welche ätherische Öle und/oder die Wirkstoffe davon und Glykolderivate als Lösungsmittel der ätherischen Öle und der Wirkstoffe davon umfasst, wobei der Docht eine Temperatur von weniger als 100 °C in seinem oberen Bereich erreicht,
**dadurch gekennzeichnet dass**
die eingesetzten Glykolderivate Glykolether sind.

2. Verfahren zum Desinfizieren, Erfrischen und Parfümieren der Luft nach Anspruch 1,
**dadurch gekennzeichnet, dass**
die ätherischen Öle bevorzugt gewonnen werden aus dem Teebaum, den Blättern des Ceylon-Zimtbaums, von Zitronellgras, Artemisia, Zitronengras, Zeder, Nelken, Kiefer, Bergamotte, Lavandin oder Mischungen davon; und dass die bevorzugt eingesetzten Wirkstoffe aus einer Gruppe ausgewählt werden aus Thymol, Zitronellal, Zitronellol, Estragol, Geranylacetat, Eukalyptol, Eugenol, Terpineol, Cinnamaldehyd, Zimtsäure, Citral, Dihydromyrcenol, Rosenoxid, Cajeputen und Mischungen davon.

3. Verfahren zum Desinfizieren, Erfrischen und Parfümieren der Luft nach einem der vorausgehenden Ansprüche,
**dadurch gekennzeichnet, dass**
die desinfizierende Zusammensetzung aromatische Verbindungen enthält.

## Revendications

1. Procédé pour désinfecter, rafraîchir et parfumer l'air basé sur les huiles essentielles et/ou des ingrédients actifs de celles-ci, ledit procédé consistant à produire par évaporation, au moyen d'une mèche chauffée indirectement, une émission de particules dans l'air d'une composition désinfectante, rafraîchissante et parfumante, qui comprend des huiles essentielles et/ou des ingrédients actifs de celles-ci et des dérivés de glycols comme milieu de dissolution des huiles essentielles et des ingrédients actifs de celles-ci, dans lequel la mèche atteint une température inférieure à 100°C dans sa zone supérieure, **caractérisé en ce que** les dérivés de glycols employés sont des éthers de glycols.

2. Procédé pour désinfecter, rafraîchir et parfumer l'air selon la revendication 1, **caractérisé en ce que** les huiles essentielles sont obtenues de préférence à partir de l'arbre à thé, à partir des feuilles de cannelle de Ceylan, à partir de citronnelle, d'armoise, de lemon-grass, de cèdre, de clous de girofle, de pin, de bergamote, de lavande ou à partir de mélanges de ceux-ci ; et les ingrédients actifs employés de préférence sont sélectionnés parmi le thymol, le citronellal, le citronellol, l'estragol, l'acétate de géranyl, l'eucalyptol, l'eugénol, le terpinéol, l'aldéhyde cinamique, l'acide cynamique, le citral, le dihydromyrcénol, l'oxyde de rose, le limonène et des mélanges de ceux-ci.

3. Procédé pour désinfecter, rafraîchir et parfumer l'air selon les revendications précédentes, **caractérisé en ce que** la composition désinfectante inclut des composés aromatiques.
